# EUROPEAN PATENT APPLICATION

(11) **EP 2 458 385 A1**
(43) Date of publication of application: **30.05.2012**
(21) Application number: 09847515.5
(22) Date of filing: 23.07.2009
(51) Int. Cl.: G01N 33/577

(54) **KIT AND METHOD FOR THE PRE-MORTEM IN VITRO DETECTION OF ALZHEIMER DISEASE**

(30) Priority: 23.07.2009 ES 200901632
(71) Applicant: Universidad Complutense De Madrid, 28040 Madrid (ES)
(72) Inventor: SANCHEZ RAMOS, Celia, 28037 Madrid (ES)
(74) Representative: Temino Ceniceros, Ignacio
(86) International application number: PCT/ES2009/000392
(87) International publication number: WO 2011/009967

(57) **Abstract**

The invention relates to a kit and method for the pre-mortem in vitro detection of Alzheimer's disease. The invention relates to a kit for detecting Alzheimer's disease by the *in vitro* identification of the presence of biomarkers in waste from crystalline lens surgery. The kit enables the pre-mortem detection of the disease, even before the appearance of clinical symptoms. The beta-amyloid peptide is selected from among the biomarkers.

## Description

### Object of the invention

The present invention relates to the early detection of Alzheimer's disease (AD) through the waste of crystalline lens surgery. Until now, the only method of reliable diagnosis of this disease consist of the analysis of brain tissue of the patient after his/her death. This invention discuss the early detection of Alzheimer's disease patients, preferably before that they show signs of the neuropathy.

### State of the art

Alzheimer's disease is a neurodegenerative disease that is manifested with cognitive impairment and behavioral disorders. It is characterized in its typical form by a progressive loss of memory and other mental abilities, as neurons die and different areas of the brain are atrophied.

The impact of this disease is such that it is estimated between 18 and 22 million people affected worldwide, with an average prevalence of 3-15% and annual incidence between 0.3-0.7%. The prevalence is variable, but it is estimated that 1-5% of the population over 65 years of age and between 20-40% of the population over 85 years suffers the disease. In fact, their prevalence doubles every five years after the age of 65 and is 50% to 60% of demented syndromes in studies post-mortem. (J. Vilalta-Franch, S. López-Pousa, J. Garre-Olmo, A. Turón Estrada e I. Pericot-Nierga. Heterogeneidad clínica de la enfermedad de Alzheimer según la edad de inicio. Revista de Neurologia 2007; 45: 67-72).

Although there is currently no cure for this disease, now available drugs to treat Alzheimer's disease can help maintain the mental abilities of patients for months or years, but not change the fundamental course of disease. In this sense, early diagnosis of the disease increases the chance of successful treatment.

However, there is currently no a method for specific and determining diagnosis for this disease, but it is based, first, on the history of the patient and the clinical observation of neurological and psychological characteristics from both the healthcare professional and members of the family. Secondly, cerebral imaging tests are carried out using different techniques among which can be indicate Computerized Axial Tomography (CAT), Magnetic Resonance (NMR), Positron Emission Tomography (PET), or Single Photon Emission Computed Tomography (SPECT); however, these techniques may show different signs that there is some type of dementia, without determining the final diagnosis. (Manuela Neumann, Deepak M. Sampathu, Linda K. Kwong. Ubiquitinated TDP-43 in frontotemporal lobar degeneration and amyotrophic lateral sclerosis. Science 2006; 314: 130-133).

The diagnosis of disease based on the clinical observation of the patient, neuropsychological and neurological tests, as well as the absence of an alternative diagnosis and supported in the brain scanner, has achieved to approach the certainty of the diagnosis to 85%, but the definitive diagnosis must be made with histological evidence on brain tissue obtained at autopsy, i.e., it is always a post-mortem diagnosis.

Post-mortem studies have shown that Alzheimer's disease is manifested in two brain damages. One of them is the formation of a protein deposit that forms the senile or neuritic plaques of β-amyloid peptide (Aβ), and the other consists of an entanglement of fibrils of neurons, known as neurofibrillary tangles.

The senile plaques of Alzheimer's disease are formed by a protein center surrounded by degenerated neurons, as well as glial and microglial cells. The Aβ peptide occurs normally in soluble, monomeric form and circulates at low concentrations in cerebrospinal fluid and blood. Despite the seemingly negative role in this neuropathy, this peptide participates in normal cellular functions, i.e.:
- It exercises an autocrine function and stimulates the cell proliferation.
- It promotes cell adhesion and protects neurons against oxidative damage.
- At physiological concentrations can act as a neurotrophic and neuroprotective factor.
- It is a physiological regulator of the activity of potassium (K) and calcium (Ca) ion channels in neurons and is secreted by some of these cells in response to neural activity to negatively regulate the excitatory synaptic transmission.
- Its deposits can catch potentially dangerous metal ions. (Selkoe DJ. Cell biology of the amyloid β-protein precursor and the mechanism of Alzheimer's disease. Annu Rev Cell Biol (1994); 10: 373-40).

The neurofibrillary tangles are remains of damaged microtubules (microtubules form the structure that allows the flow of nutrients through the neuron). The formation of these tangles begins in the region of the hippocampus in which resides the function of the memory management and the breakdown of the microtubular system originates defects from axonal transport and probably cellular degeneration, which has been observed in the neurons affected by Alzheimer's disease. The tau proteins, which belong to the family of the "Microtubules-Associated Protein" or MAP are involved in this process, whereby many times the diseases in which is observed the formation of neurofibrils are called tauopathies (Castaño, E.M; Frangione. Biology of disease: Human amyloidosis, Alzheimer's disease and related disorders. Laboratory Investigation (1998) 58: 122).

In the cytoplasm, the tau protein is usually phosphorylated, i.e. has joined phosphate groups, which are very important in regulating the function of the protein. This phosphorylation allows the mobility of the protein tau into the axons of neurons in the centrifugal direction. The helical filaments are composed of tau aggregates which, unlike the normal protein, contain a large number of phosphates by which it is said that tau is hyperphosphorylated (Wang H. Y., Li W., Benedetti N. J. and Lee D. H. Alpha 7 nicotinic acetylcholine receptors mediate beta-amyloid peptide-induced tau protein phosphorylation. J. Biol. Chem. (2003) 278, 31: 547-31 553).

In Alzheimer's disease and other tauopathies there is a phenomenon of hyperphosphorylation and/or abnormal phosphorylation. It has been shown that neither soluble form of hyperphosphorylated tau nor that which forms the filaments has capacity to promote the formation of microtubules or stabilize microtubules previously formed. Moreover, this form of tau inhibits tubulin assembly in the microtubules and can disarm those formed with the normal protein.

For several years it has been studied the detection of different biomarkers (biological markers) in several body fluids, mainly in cerebrospinal fluid, blood and urine, as basis of future diagnostic tests for the detection of the AD, for the moment, however, it has not possible for the skills in the art to establish any protocol of use of a biomarker in the pre-mortem diagnosis of AD in this type of samples (H.M. Schipper. The role of biologic markers in the diagnosis of Alzheimer's disease. Alzheimer's & Dementia (2007) 3: 325-332). In this sense, the Aβ peptide and the tau protein are the main biomarkers for the in vitro diagnosis of AD.

The patent EP1420830A1 describes a method of ocular diagnosis in vivo of the AD by the use of compounds or markers that bind to the peptide Aβ present in ocular tissues of the patients. The markers are usually fluorophore compounds which when are bound to the peptide Aβ allow to detect and quantify the present Aβ by means of measurements of fluorescence, which would put in evidence the existence or not of the disease in the patient. The procedure disclosed consists of applying these markers in the eye in the form of liquid or gel. The lipophilicity of these compounds facilitates the penetration in eye tissue, where bind to the Aβ peptide. Later, after a period of time to ensure the penetration of the compounds in eye tissue and the binding to the peptide Aβ, the measurement of fluorescence is carried out directly in the eyes of the patient. It is, therefore, a pre-mortem, in vivo and non-invasive diagnosis of AD.

On the other hand, the patent EP1913866A1 discloses a new non-invasive way for detecting biological markers of AD in the crystalline lens and other ocular tissues using quasi-elastic light scattering, Raman Spectroscopy, fluorometry or other optical techniques. These techniques allow the detection and monitoring of deposition of Aβ peptide in the eye for the diagnosis of neurodegenerative diseases such as AD.

However, despite the efforts and progress described above, the definitive diagnosis of AD, at the moment, still is carrying out only post-mortem. It is still essential, therefore, to develop new tools for early and preferably preclinical detection, of the AD, which enable to establish the suitable treatment in the early disease stages, so that it can delay the development of the symptoms of the same.

### Description of the invention

One aspect of the present invention relates to a kit of detection of the AD using the waste from crystalline lens surgery where the ocular waste is extracted. In that waste the presence of a biomarker of the AD, preferably the Aβ peptide, is analyzed, which, in addition to be deposited in the brain of Alzheimer's sufferers giving rise to the senile or neuritic plaques, also makes it in the crystalline lens.

Another aspect of the invention refers to the use of the waste from crystalline lens surgery in the development of a method for the detection of the AD by detecting a biomarker, for example by the detection of Aβ peptide.

In ophthalmic medicine, the total or partial opacification of the crystalline lens is called cataracts. Today, the surgical intervention of crystalline lens more frequent is the surgery of cataract. The cataract is a chronic disease associated with the aging process and the gradual increase in life expectancy has caused a substantial increase in the prevalence of cataracts that affects an increasing proportion of the population. Cataracts can be developed for several reasons, however, acquired cataract is the most common type and it is the main cause of vision loss among older than 55 years, thereby that cataract surgery is a very common intervention at age ranges susceptible of suffering from Alzheimer's disease. In fact, in people younger than 50-55 years the prevalence of cataracts is low, in the order of the 0.2% to 7%; in groups of intermediate ages, between approximately 55 and 65 years, cataracts affect to about 20% of the population of that age; and, from the age of 70-75, cataracts affect between the 40% and more than 60% of the population. Still without producing serious vision problems which force a surgery, more than 75% of people older than 60 years and 95% of people older than 75 already present some degree of opacity in the lens.

Among surgeries carried out for the removal of cataracts, currently the most widespread is extracapsular surgery, since it has fewer complications than other techniques and allows the use of an intraocular lens. Through this surgery only the opaque portion of the cataract is removed and the posterior portion of the capsule is left, which provides support for an intraocular lens that occupies the same place as the crystalline lens extracted. Extracapsular surgery is usually performed using a technique called phacoemulsification in which a small incision in the eye tissue is performed, followed by the destruction of the opaque lenses of the patient by applying ultrasonic waves that produce a vibration between 30000 and 60000 times per second. This vibration breaks down the cataract into fragments sufficiently small to be emulsioned and gently drawn in. Phacoemulsion apparatus consist of a system of serum circulation with irrigation and aspiration, connected to the ultrasound terminal itself. This system allows drawn in emulsified fragments at the same time that maintains the terminal of the emulsifier refrigerated to prevent bums. The fragments of opaque lens are collected together with the serum in a container and constitute the waste from crystalline lens surgery. To complete the operation, a lens is inserted into the place that occupied the crystalline lens of the patient (Olitsky SE, Hug D, Smith LP. Abnormalities of the Lens. In: Kliegman RM, Behrman RE, Jenson HB, Stanton BF, eds. Nelson Textbook of Pediatrics. 18a ed. Philadelphia, Pa: Saunders Elsevier; 2007: cap. 627).

The kit of the invention for detecting the AD includes two components. First, it has a container element where the waste from crystalline lens surgery is collected. These remains are in a liquid that usually is saline solution. Secondly, the kit contains a marking element that binds to the biomarker from AD in the whole of the remains of the crystalline lens. If necessary, the kit includes a system for detecting the marking element joined the biomarker, that allows to check the presence or not of said biomarker and, therefore, the AD in the patient.

In addition, the kit may contain a system to separate the solid part and the liquid part from remain mixture obtained as a result of the surgery. This is usually a centrifuge, which prints to the mix a rotary movement with a greater intensity than the gravity force, causing the sedimentation of the solid remains in question.

The waste from crystalline lens surgery that is included in the kit allows detecting the content of biomarker in the crystalline lens of the patient as result of the onset and development of the AD. The present invention also refers to the method of *in vitro* detection of a biomarker from AD in the crystalline lens and this method includes, first of all, the collection, in a container element of the remains of crystalline lens extracted during the cataract surgery together with the serum used in the same. This mixture can be treated directly with elements of marking that bind to the biomarker, or may be centrifuged to separate the liquid part of the mixture, corresponding to the serum, from the solid part, corresponding to the fragmented cells. Once separated, the solid part is processed for the marking of the selected biomarker from AD to detect the disease in the patient. This step varies depending on the technique selected for the biomarker detection. The technique can be characterized by the inclusion of the sample in paraffin blocks from which cuts or slices are subsequently obtained on which the marking element of the selected biomarker from AD is applied. These blocks can also be obtained by rapid freezing of the solid part of the sample. Another option is to separate proteins from the rest of elements of the solid part by any of the usual techniques (sonication, treatment with mild detergent, etc.) and, subsequently, detect the presence or absence of the biomarker in the mixture of proteins by means of any of the techniques used by the skills in the art (Western blot, ELISA immunoenzymatic assays, microarrays of proteins, etc.) and using for this the appropriate marking element.

In the present invention the term "marking element" means any compound that shows specifically the presence of a particular component of a heterogeneous mixture and, more specifically, when the component is a biomarker; and the term "biomarker" means a substance, whose presence is objectively measurable and assessable, used as indicator of the existence of the AD either by its own presence or changes in quantity or concentration.

The biomarker used in this invention can be any of the biomarkers from AD. Preferably it is used the Aβ peptide, that means proteins or peptides which form part of neuritic or senile plaques, both the amyloid protein and the p-amyloid precursor protein (APP) or any of the isoforms of between 39 and 42 amino acid remains generated by the natural proteolytic process which gives rise to several peptides (Aβ₁₋₄₀, Aβ₂₋₄₀, Aβ₁₋₄₂) from the APP.

The marking element selected varies depending on the technique chosen to detect the presence of AD. Frequently, these markers already known in the state of the art are fluorophore compounds, i.e. compounds that emit fluorescence once attached to the biomarker, which allows detecting the presence of this biomarker in the sample in question. For example, a fluorophore widely used for the detection of beta-amyloid is the Congo red or derivatives thereof, which gives rise to the emergence of deposits of amyloid stained in tone between pink and orange, easily identifiable. As well as the use of other fluorophores, the detection of plaques can be made with other compounds such as thioflavin, violet Crystal, or silver methenamine. Specific anti-biomarker antibodies can also be used as elements of marking using conventional techniques.

The kit and method of the invention are a benefit for the patient, since its use enables the *in vitro* detection of a biomarker from AD and not directly on the eye itself, by which is not necessary to apply any gel or ointment with the marking elements of Aβ on the eye of the patient or make measurements directly on it, as it is the case with any of the last known inventions for detecting Aβ (EP1420830A1), thus avoiding any side effects, such as anaphylactic reactions. On the other hand, in the elaboration of the method and kit for detection of the AD, the waste from crystalline lens surgery and which are systematically discarded are used providing it a utility to analyze the presence of Aβ or other biomarkers from AD. Given that there are carried out crystalline lens surgery in people of similar age or even prior to the onset of clinical symptoms of AD in which remains of crystalline lens and other ocular fluids are obtained, the present invention provides tools for the early detection of this disease so that early treatments which mitigate or delay the progression of the disease can be established.

### Embodiment of the invention

The following examples that accompany help to a better understanding of the invention and have illustrative, and non-limitative, character of it.

### Example 1. Sampling

The waste removed in lens surgery of 42 patients, aged between 68 and 88 years, through phacoemulsion is utilized. Said wastes had been collected in plastic waste bags, usual in this type of surgery, and each one of them, contained the waste of crystalline lens and other waste of ocular fluids of a surgical operation together with the physiological serum used in the same. Samples were kept at 4 ° C until the time of processing.

### Example 2. Separation of the solid remains of crystalline lens

In the first place the dilution of cellular debris and physiological saline solution was extracted from the bag and was left in a beaker for 24 hours to produce a natural precipitation of solid remains, passed this time, all the possible supernatant was removed followed by the centrifugation of the supernatant. A volume of 15 ml was extracted from the supernatant of this first centrifugation, which was designated dissolution M1, and, in a test tube, it was centrifuged for 15 minutes at 5000 revolutions per minute. This operation provided a mass of solid remains and a dissolution that was designated M2.

From this new dissolution M2 was extracted a volume of 5 ml and was centrifuged in an Eppendorf tube for 10 minutes at 5000 revolutions per minute. This operation provided a mass of solid remains and a dissolution that was designated M3.

From this new dissolution M3 was extracted a volume of 5 ml and was centrifuged in an Eppendorf tube for 5 minutes at 5000 revolutions per minute. This operation provided a mass of solid remains and a dissolution that was designated M4.

And finally from the dissolution M4 was extracted a volume of 5 ml and was centrifuged in an Eppendorf tube for 5 minutes at 5000 revolutions per minute. This operation provided a mass of solid remains and a supernatant that was discarded.

All the mass of solid remains obtained by the different centrifugations was put together and a last centrifugation to remove all the supernatant as possible from the mass of solid remains was undertaken, obtaining a compact mass.

### Example 3. Inclusion in paraffin of the mass of solid remains extracted from the dissolution in serum of lens fragments

Once obtained the mass of solid remains was included in a "well" to which both ends was covered with a fine cloth or stocking. The wells were placed in a container with running water and the open faucet for washing during a time of 2 to 4 hours. Once done the washing the dehydration of the mass in alcohol was undertaken.

The sample was extracted from wells, dried with sterilized gauze and included in 30% alcohol for 30 minutes. Once passed this time, the sample was dried with sterilized gauze and the inclusion in 50% alcohol for 25 minutes was undertaken. After the 25-minute inclusion in 50% alcohol, the drying of the sample and subsequent inclusion in 70% alcohol for 24 hours were undertaken.

The sample was dried with sterilized gauze and the inclusion in 100% alcohol was undertaken. This inclusion was carried out in two steps of 25 minutes each. The drying of the sample with sterilized gauzes was undertaken and it was included in 100% alcohol. This inclusion was carried out in three steps, two of them of 25 minutes and the last one of 1 hour and 30 minutes. The sample was dried and its inclusion in xylene for 40 minutes was undertaken.

Dissolution of liquid paraffin and xylene was prepared with a ratio 1:1 and the sample was included in said solution for 30 minutes in an oven to 50 °C. Passed this time the sample was included in liquid paraffin in three steps, two of them of 45 minutes and a final step of 1 h and 30 minutes, during this phase the containers with paraffin were inside a stove at a temperature of 50 °C.

Once completed this dehydration process the assembly of paraffin blocks was began. In order to mount the paraffin blocks were available liquid paraffin at a temperature of 50 °C, Leuckart bars and a Bunsen burner. Leuckart bars were placed on a metal surface, paraffin was poured, the identification of the sample was placed, and the inclusion of the sample in the block still liquid was undertaken. Once completed this operation the block was introduced into a crystallizer with water running until it solidified with which the paraffin with the included sample block was achieved.

Sections of the paraffin block of 10 mm thickness were obtained using a microtome. Once obtained a series of cuts, they were deposited on water at 40 °C so that the cuts were stretched. Next the cuts were collected with slides placed in a stove at 370 °C overnight to fix them.

### Example 4. Staining with Congo red in Mayer's hematoxylin solution

Once cut, the rehydration was undertaken to allow its staining following the reverse process to the dehydration described in example 3, i.e., using alcohol each time at less concentration until leave the cuts in deionized water.

The staining was then undertaken in Mayer's hematoxylin solution for 10 minutes, rinsed for 5 minutes with tap water and left in alkaline sodium chloride solution for 20 minutes. It was then stained with alkaline Congo red solution (SIGMA-ALDRICH) for 20 minutes, following the manufacturer's instructions.

By staining with Congo red and using a doubly polarized light microscope, the appearance of a coloring was used as a criterion to determine the presence of peptide Aβ in the sample.

### Example 5. Staining with Congo red in Gill's hematoxylin

It was carried out as example 4, but instead of Mayer's hematoxylin was used Gill's hematoxylin No. 3 and it was stained for 3 minutes.

### Example 6. Staining with thioflavin T

Once cut, the rehydration was undertaken to allow its staining following the reverse process to the dehydration described in example 3, i.e., using alcohol each time at less concentration until leave the cuts in deionized water.

The cuts already deparaffinized and hydrated were incubated in humidity chamber at ambient temperature for 2 hours with the anti-peptide serum. Subsequently the were incubated with a mixture of IgG anti-rabbit IgG conjugated with Alexa-594 diluted 1:500 and 10 µM thioflavin T, both prepared in 1% (p/v) bovine serum albumin (BSA) in 1X PBS buffer pH 7.4; for 4 hours in darkness. The cuts were then washed for 5 minutes three times in 1X PBS/0.05% Tween 20 (p/v).

The cuts were mounted in the medium for fluorescence and were observed in the epifluorescence microscope using filters for rhodamine (594 nm) and FITC (488 nm) to visualize the red fluorescence of Alexa-594 and green fluorescence of thioflavin T.

### Example 7. Assembly

After staining, the cut was dehydrated and the rinsing and the final assembly were undertaken. Dehydration was carried out with alcohols with increasing graduations.

The rinsing was made with xylol. The aim of this step is to soak the cut with the Canada balsam solvent, which at the same time confers it a glass-like refractive index.

For mounting the slide around the cut was cleaned and deposited on it a drop of Canada balm dissolved in xylol and it was covered with a coverslip. It was left to dry a few hours before its observation with the microscope.

### Example 8. Detection of Aβ peptide in paraffin cuts by monoclonal human anti-Aβ antibodies

Monoclonal Mouse Anti-Human Beta-Amyloid (Dako) was used, at dilutions of 1:50, and applied to sections included in paraffin and fixed in formalin, using 10 minutes of epitope recovery by heat and incubation for 30 minutes at room temperature with the primary antibody.

For the recovery of the epitope, the tissue cuts were deparaffinized and rehydrated as described in example 4, and the cuts were immersed, which were at room temperature in a preheated solution and diluted 1:10 with deionized water by Dako Target Retrieval Solution, High pH 10x concentration, in a water bath at 95 °C. They were incubated for 30 minutes. The container with the slide of the water bath was removed.

Once cooled the sample for 20 minutes at room temperature the Target Retrieval Solution, High pH was decanted and the cuts were rinsed three times with buffer at pH 8.0 containing 5.0 M guanidineHCI and 50 mM trisHCI, at room temperature.

Negative control was carried out with DakoCytomation Mouse IgGI, diluted at the same concentration of murine IgG than the primary antibody.

### Example 9: Detection of Aβ Peptide by an immunoenzymatic technique

Aβ-peptide levels were quantified by sandwich ELISA using a commercial kit, Innotest A β1-42, (Innogenetics, Belgium), which detects fragment β1-42 of amyloid.

The sample obtained according to example 2 was solubilized in a buffer solution chilled on ice containing 5.0 M guanidineHCI and 50 mM trisHCI, pH 8.0; the samples were in incubation for 3 to 4 hours and then diluted 1:10 in buffer solution chilled on ice with 0.25% casein, 0.05% sodium azide, 20 µg/ml of aprotinin, 5 mM EDTA, pH 8.0, 10 µg/ml of leupeptin in PBS. They were centrifuged at 16,000*g* for 20 minutes at 4 °C.

There were used wells impregnated with primary antibody (anti-Aβ) which were incubated for 1 hour at 37 °C with another biotinylated anti-Aβ antibody, the corresponding samples, streptavidin conjugated with peroxidase and chromogenic solution (tetramethylbenzidine dissolved in dimethyl sulfoxide). The reaction was quenched with IN sulfuric acid and the absorbance of each well was read with a reader at a wavelength of 450 nm.

It was performed a standard curve between 100 and 2,000 pgml which allowed to establish the equation and transform the data of absorbance at protein concentration (pg/ml). For each sample the determination was doubled and the average of both was taken as final result. Those samples in which the determining difference between the two wells was more than 20% were discarded.

## Claims

1. Kit for detecting Alzheimer's disease comprising:
a) a container element where the eye waste extracted during crystalline surgery is collected.
b) a marking element that binds to a biomarker for Alzheimer's disease that is present in eye waste collected in the container element;

2. Kit for detecting Alzheimer's disease, according to the claim 1, comprising, in addition, a detecting system of the marking element bound to the biomarker.

3. Kit for detecting Alzheimer's disease, according to any of the claims 1 and 2, comprising further the necessary means to carry out the separation of the solid part and liquid part of the content from container element.

4. Kit for detecting Alzheimer's disease, according to any of the preceding claims, wherein the biomarker is the β-amyloid peptide.

5. Kit for detecting Alzheimer's disease, according to any of the preceding claims, wherein the marking element is a fluorescent compound.

6. Kit for detecting Alzheimer's disease, according to the claim 5, wherein the fluorescent compound is Congo red or derivatives thereof.

7. Kit for detecting Alzheimer's disease, according to the claim 5, wherein the fluorescent compound is thioflavin T.

8. Kit for detecting Alzheimer's disease, according to any of the claims 1-4, wherein the marking element is an antibody.

9. Kit for detecting Alzheimer's disease, according to the claim 8, wherein the antibody is an anti-beta-amyloid monoclonal antibody.

10. Kit for detecting Alzheimer's disease, according to any of the claims 1, 2, 3, 4, 8, and 9, wherein the detecting system is based on an immunological technique.

11. Kit for detecting Alzheimer's disease, according to the claim 10, wherein the immunological technical is selected from sandwich-type ELISA, competitive ELISA, direct ELISA, indirect ELISA, and Western Blot.

12. *In vitro* method for detecting Alzheimer's disease comprising:
a) recovering the waste from a crystalline lens surgery;
b) detecting a biomarker from Alzheimer's disease in the recovered waste.

13. *In vitro* method according to the claim 12 including an additional step c) between the step a) and step b), and wherein said additional step c) comprises marking the biomarker from Alzheimer's disease with a marking element.

14. *In vitro* method according to any of the claims 12 and 13 including an additional step d) between the step a) and step b) or either between the step a) and step c) and wherein said additional step d) comprises separating the solid and liquid part from the recovered waste.

15. *In vitro* method according to any of the claims 12-14, wherein the biomarker is the beta-amyloid peptide.

16. *In vitro* method according to any of the claims 13-15, wherein the marking for the step c) is carried out with a fluorescent compound.

17. *In vitro* method according to the claim 16, wherein the fluorescent compound is Congo red or derivatives thereof.

18. *In vitro* method according to the claim 16, wherein the fluorescent compound is thioflavin T.

19. *In vitro* method according to any of the claims 13-15, wherein the marking of the step c) is carried out with an antibody.

20. *In vitro* method according to the claim 19, wherein the antibody is an anti-beta-amyloid monoclonal antibody.

21. *In vitro* method according to any of the claims 12, 13, 14, 15, 19, and 20, wherein the detecting system is based on an immunological technique.

22. *In vitro* method according to the claim 21, wherein the immunological technique is selected from sandwich-type ELISA, competitive ELISA, direct ELISA, indirect ELISA, and Western Blot.

23. Use of waste from lens surgery in the development of a method *for in vitro* diagnosis of Alzheimer's disease.

24. Use according to the claim 23 wherein the method *for in vitro* diagnosis comprises:
a) recovering the waste from a crystalline lens surgery;
b) detecting the presence or absence of a biomarker from Alzheimer's disease in the recovered waste.

25. Use according to the claim 24 wherein the method *for in vitro* diagnosis includes an additional step c) between the step a) and step b) and wherein said additional step c) comprises marking the biomarker from Alzheimer's disease with a marking element.

26. Use according to any of the claims 24 and 25 wherein the method *for in vitro* diagnosis includes an additional step d) between the step a) and step b) or either between the step a) and step c) and wherein said additional step d) comprises separating the solid part and liquid part from the recovered waste.

27. Use according to any of the claims 24-26, wherein the biomarker is the beta-amyloid peptide.

28. Use according to any of the claims 23-27, wherein the marking of the step c) is carried out with a fluorescent compound.

29. Use according to the claim 28, wherein the fluorescent compound is Congo red or derivatives thereof.

30. Use according to the claim 28, wherein the fluorescent compound is thioflavin T.

31. Use according to any of the claims 23-27, wherein the marking of the step c) is carried out with an antibody.

32. Use according to the claim 31, wherein the antibody is an anti-beta-amyloid monoclonal antibody.

33. Use according to any of the claims 24, 25, 26, 27, 31, and 32, wherein the detecting system is based on an immunological technique.

34. Use according to the claim 33, wherein the immunological technique is selected from sandwich-type ELISA, competitive ELISA, direct ELISA, indirect ELISA, and Western Blot.
